Europäisches Patentamt

European Patent Office

Office européen des brevets

(11) Numéro de publication: **0 177 477**
**A2**

(12) **DEMANDE DE BREVET EUROPEEN**

(21) Numéro de dépôt: **85870127.9**

(22) Date de dépôt: **20.09.85**

(51) Int. Cl.⁴: **C 12 P 19/14**

(30) Priorité: **26.09.84 BE 900679**

(43) Date de publication de la demande:
**09.04.86 Bulletin 86/15**

(84) Etats contractants désignés:
**AT BE CH DE FR GB IT LI LU NL SE**

(71) Demandeur: **Région wallone, répresentée par l'Exécutif régional wallon**
**Boulevard de l'Empereur, 11**
**B-1000 Bruxelles(BE)**

(72) Inventeur: **Thonart, Philippe**
**Rue de la Houlette, 20**
**B-5850 La Bruyere(BE)**

(72) Inventeur: **Artois, Claude**
**Rue des Cottages, 123**
**B-1180 Bruxelles(BE)**

(74) Mandataire: **Blanchart, André**
**MINISTERE DE LA REGION WALLONNE Direction de l'Energie et des Technologies Nouvelles Place Joséphine Charlotte 3 (Boites 27-28)**
**B-5100 Jambes Namur(BE)**

(54) **Procédé d'hydrolyse enzymatique de solutions aqueuses d'inuline.**

(57) Procédé d'hydrolyse enzymatique de solutions aqueuses d'inuline par des cellules microbiennes possédant une activité inulinase, caractérisée en ce que les cellules microbiennes sont soumises à un traitement thermique de perméabilisation des parois cellulaires à une température et une durée telles que toutes les cellules sont tuées.

EP 0 177 477 A2

1

PROCEDE D'HYDROLYSE ENZYMATIQUE DE SOLUTIONS AQUEUSES D'INULINE

La présente invention a pour objet un procédé d'hydrolyse de solutions aqueuses d'inuline par des cellules microbiennes à activité inulinase, éventuellement immobilisées sur un support solide en vue d'obtenir des sirops de fructose.

Les sirops de fructose sont de plus en plus utilisés dans l'industrie alimentaire en remplacement de saccharose. Le fructose a un plus grand pouvoir sucrant que le saccharose et le glucose et de plus, présente diverses qualités telles que pas de dépendance vis-à-vis de l'insuline et une faible caryogénicité.

A côté de ces avantages diététiques, le fructose est fréquemment utilisé dans les industries pour diverses propriétés technologiques : citons par exemple, la limitation de la déshydratation et de la cristallisation, les propriétés antioxydantes. Dans les aliments acides, le fructose est préféré au saccharose car celui-ci peut s'hydroliser.

De plus, l'inversion du saccharose par voie acide ou enzymatique donne un mélange équimoléculaire de glucose et de fructose. La production de sirops contenant du fructose est réaliséeégalement par l'isomérisation du glucose en fructose par la glucose isomérase. Ces sirops sont appelés isomérose et sont produits à partir d'un hydrolysat d'amidon en utilisant une enzyme immobilisée. Des techniques chromatographiques ont été développées pour enrichir en fructose ce sirop et atteindre des concentrations en fructose de 90 %.
Une voie alternative est la production d'une D-glucosone à partir de glucose en utilisant une pyranose 2-oxydase suivi d'une réduction catalytique en fructose.
Il est également possible de produire des sirops de fructose à partir de polymères de fructose appelés inuline qui est présente notamment dans les tubercules de dahlia, de chicorée et de topinambour.
L'inuline est un polymère de fructose non ramifié qui comporte dans sa forme naturelle, 35 unités fructose terminées par une unité glucose enchaînée dans une liaison $\alpha$ 1, $\beta$ 2 comme dans le saccharose.
L'inuline peut être extraite des plantes au moyen d'eau chaude par des méthodes bien connues telles la diffusion à contre-courant des cossettes à des températures élevées ou en pressant le jus de la plante.

Le jus cru peut être purifié par filtration à travers du charbon actif ou traité par de la chaux avec un procédé fort semblable à celui de la sucrerie de betterave. Les polymères extraits peuvent avoir des longueurs de chaînes variables et ontun degré de polymérisation de 5 à 15.

L'inuline peut être hydrolysée par voie acide dans des conditions relativement peu rigoureuses,pH 1-2, 1-2 heures à 80-90°C.
Ce procédé est peu spécifique et provoque une coloration ainsi que la formation de produits secondaires.

Suivant un autre procédé plus spécifique que le procédé acide, une hydrolyse enzymatique peut être réalisée.

De nombreux microorganismes sont connus comme producteurs d'enzymes, hydrolysant l'inuline, appelés inulinases. Citons par exemple , Kluyveromyces fragilis, Candida pseudotropicalis ou Kephyr, Pichia polymorpha, des espèces d'Aspergillus,de Fusarium,de Penicillium, d'Achromobacter et d'Acetobacter.

Les inulinases microbiennes sont produites de manière intra-et extracellulaire notamment dans le cas de Kluyveromyces fragilis. Les conditions de culture peuvent influencer la proportion des enzymes intra-et extracellulaire. De plus, il est connu qu'il y a 2 activités enzymatiques qui composent l'activité inulinase.
Ces deux activités sont dénommées endoinulisases et exoinulisases.
Dans le brevet belge n° 893013 de la Société NOVO Industry A/S, "Procédé de production de fructose à partir de l'inuline au moyen d'une nouvelle préparation d'inulinase et préparation à cet effet", la Société NOVO définit les proportions qui doivent exister entre ces endo- et exoinulinases pour hydrolyser l'inuline à partir d'enzymes de l'organismes Aspergillus.

Ce procédé présente toutefois l'inconvénient de ne pas récupérer l'enzyme après utilisation et la mise en oeuvre d'un procédé continu est délicat. A côté du procédé utilisant les enzymes à l'état libre, on a développé des procédés d'immobilisation des enzymes et/ou des cellules microbiennes les contenant.

L'utilisation de cellules entières présente de multiples avantages comparativement au procédé utilisant les enzymes libres ou immobilisées à condition que le substrat puisse pénétrer dans la cellule et/ou être en contact avec l'enzyme. De plus,l'extraction et la purification des enzymes ne sont pas nécessaires et les deux activités inulinases sont disponibles en même temps.

GUIRAUD J.P.; BAYON A.M.; CHAUTARD P. et P. GALZY, Enzyme Microbiol. Technol., 1983, 5, 185-190, ont mis au point un réacteur à cellules immobilisées qui hydrolyse en continu une solution d'inuline en fructose et glucose. La souche utilisée est Pichia polymorpha et les conditions de réaction sont telles qu'elles minimisent le développement cellulaire ainsi que la formation de métabolites secondaires (tel que l'éthanol). Le temps de vie du réacteur est de l'ordre de 75 jours. Suivant ce procédé, les cellules sont maintenues vivantes et consomment de ce fait du substrat pour leurs réactions secondaires. De plus, les conditions de réaction sont délicates à réguler.

La présente invention remédie à ces divers inconvénients et convient plus particulièrement à des procédés continus d'hydrolyse enzymatique des solutions d'inuline.

Suivant l'invention, le procédé d'hydrolyse enzymatique de solutions aqueuses d'inuline par des cellules microbiennes possédant une activité inulinase est caractérisé en ce que les cellules microbiennes sont soumises à un traitement thermique de perméabilisation des parois cellulaires pendant une durée telle que toutes les cellules sont tuées.

Le traitement thermique de perméabilisation des parois cellulaires des cellules microbiennes a pour effet notamment d'améliorer le transfert d'inuline et permettre des rendements élevés d'hydrolyse. Ce traitement thermique s'effectue à une température et pendant une durée telles que les cellules sont tuées. En général, une durée de 15 minutes au maximum suffit pour tuer toutes les cellules. Dans bien des cas, les cellules sont tuées après 5 à 10 minutes de chauffage. Ce traitement thermique détruit ainsi les activités secondaires des cellules et ces dernières ne consomment pas le fructose produit. Le traitement thermique de perméabilisation des parois cellulaires s'effectue à une température de 55°C à 75°C et pendant une durée telle que toutes les cellules sont tuées.

Pour le choix de la température, on tiendra compte du type de

cellule utilisée, de la durée du traitement de perméabilisation, éventuellement de la méthode d'immobilisation et du type de support solide utilisé. Les courbes de % d'hydrolyse en fonction du temps de solutions d'inuline à différentes températures et durées de traitement thermique des cellules permettront de faire choix des températures optimales en tenant compte des conditions opératoires fixées.

Suivant le procédé de la présente invention, les cellules microbiennes peuvent être utilisées telles quelles ou être immobilisées sur un support solide.

Si l'on fait choix de la voie par immobilisation des cellules sur un support solide, le traitement thermique de perméabilisation des parois cellulaires peut être réalisé avant ou après immobilisation des cellules microbiennes.

Tout procédé d'immobilisation peut être appliqué. On peut, par exemple, fixer les cellules microbiennes par simple adsorption sur un support solide de nature quelconque, tel que la sciure de bois, de la brique concassée, des billes de verre, etc...

Suivant une autre technique, les cellules microbiennes peuvent être emprisonnées par des substances enrobantes poreuses ou semi-perméables ou sous forme de gels tels que l'alginate de calcium, le polyacrylamide, le gel d'agar, etc...

Suivant une autre variante, les techniques de liaison par covalence (par exemple : glutaraldéhyde) peuvent être utilisées.

Les solutions aqueuses d'inuline utilisées pour l'hydrolyse peuvent être utilisées en concentrations quelconques.

En général, on utilise des solutions aqueuses d'inuline de concentration variant de quelques % à 14-15 % en inuline.

Ces solutions peuvent être mélangées en proportions variables avec des solutions aqueuses de saccharose.

Les cellules microbiennes à activité inulinase proviennent de levures, de moisisures ou de bactéries. Les cellules microbiennes provenant de levures sont choisies notamment parmi les souches de Kluyveromyces, de Saccharomyces, de Candida, et de Pichia et les cellues microbiennes provenant de moisissures et de bactéries sont choisies notamment parmi les souches de Fusarium, d'Aspergillus, de Penicillium, d'Achromobacter et de Acetobacter.

Les activités inulinases des cellules microbiennes sont déterminées par des méthodes connues. A titre d'exemple, les activités inulinases (endo et exo) sont données à partir des levures de Kluyveromyces fragilis ATCC 12424 et de Candida pseudotropicalis IP 513, dont les enzymes produites sont intra-et extracellulaires.

Le dosage de l'activité exoinulinase est réalisé par une mesure de la quantité de glucose produit après une hydrolyse d'une solution de saccharose à 10 %.

Le dosage du glucose est effectué par la combinaison d'une réaction enzymatique (glucose – oxydase) et une mesure ampérométrique à l'aide de l'anlyseur industriel de sucre YSI, modèle 27.

Les activités endo-et exoinulinase sont déterminées globalement par une estimation de la quantité de sucres réducteurs produits après une hydrolyse d'une solution d'inuline à 5 %. Le dosage des sucres réducteurs est réalisé par la méthode bien connue au DNS. Une unité inulinase est la quantité d'enzyme nécessaire pour former par minute 1 μ môle de sucre réducteur dans les conditions opératoires définies (37°C – 15 ou 30 minutes, pH 5,00).

La présente invention sera mieux comprise à l'aide des exemples non limitatifs suivants.

Exemple 1

A/ Préparation des cellules de Kluyveromyces fragilis

Les activirés inulinases (endo et exo) sont produites à partir de Kluyveromyces fragilis ATCC 12424 dont les enzymes sont intra-et extracellulaires.

Le milieu de culture utilisé contient 5 % de saccharose, 0,5 % d'extrait de levure, 1 % $(NH_4)_2SO_4$, 1 % $K_2HPO_4$ et 0,05 % de $MgSO_4$. Dans un fermenteur de 20 l, le débit d'air est de 1 VVM (12 1/min.), la vitesse d'agitation varie entre 350 et 850 t/min en fonction du taux d'oxygène dissous. Ce taux est maintenu supérieur à 40 % par rapport à la saturation en oxygène dissous. La température est régulée à 30°C. Le pH varie entre 4 et 5.

Quand la phase stationnaire est atteinte, les cellules sont récupérées par centrifugation.

L'activité enzymatique intracellulaire des inulinases (endo et exo combinées) vaut environ 300 U.I. par ml de culture.

B/ Perméabilisation des cellules de Kluyveromyces fragilis

Cette technique doit permettre d'augmenter la perméabilité cellulaire à l'inuline, tuer les cellules afin qu'elles ne consomment pas le fructose et de plus, ces cellules perméabilisées doivent maintenir une activité inulinase élevée.

La température associée à une durée de traitement a été utilisée comme agent de perméabilisation. Les cellules produites selon A, ont été chauffées 5 minutes dans un tampon acétate de Na 0,05 M pH 5 à des températures comprises entre 40°C et 80°C.

Après traitement, une suspension cellulaire (2mg/ml de matière sèche) contenant 3 % d'inuline est mise en incubation à 30°C pendant 1, 2, 3, 4 et 24 heures. Le pourcentage d'hydrolyse est mesuré par l'augmentation du pouvoir réducteur.

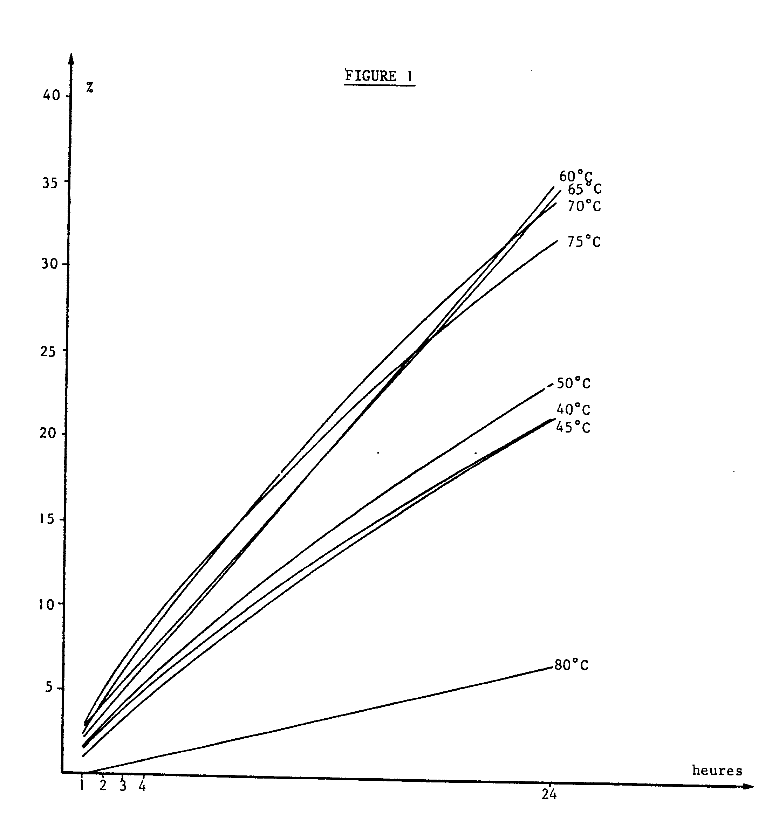

La figure 1 montre l'influence du traitement de perméabilisation à différentes températures variant entre 40° et 80°C pour une durée de traitement de 5 minutes sur le pourcentage d'hydrolyse (ordonnée) d'une solution d'inuline à 3 % en fonction du temps (abscisse).

Dans la zone comprise entre 60 et 75°C, la perméabilisation est efficace. De plus, pour ces températures maintenues pendant 5 minutes, la mortalité des cellules, déterminée au bleu de méthylène, est totale et l'activité enzymatique reste élevée.

A partir de 80°C, l'activité inulinase devient pratiquement nulle.

C/ Influence de la température de réaction sur le pourcentage d'hydrolyse obtenu à partir de cellules de Kluyveromyces fragilis, perméabilisées à 65°C pendant 5 minutes, non immobilisées.

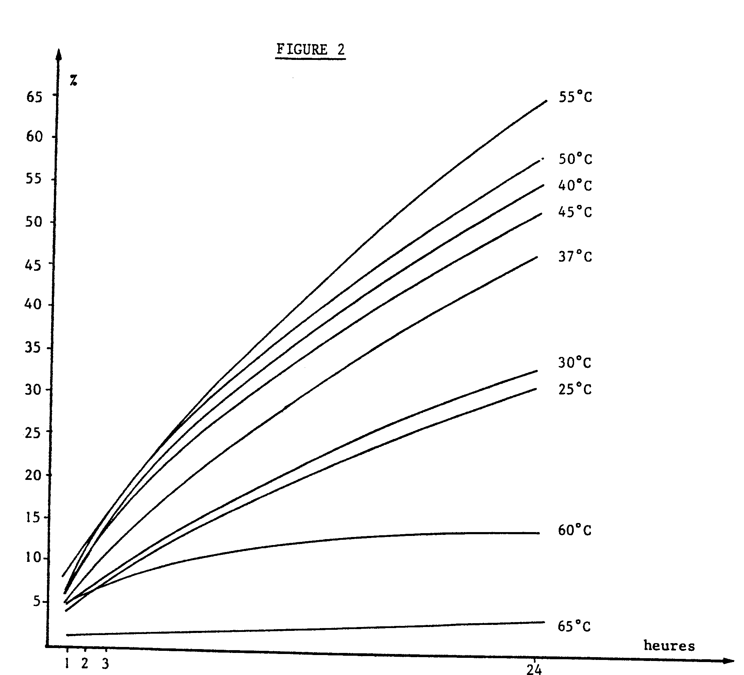

Des cellules perméabilisées à 65°C pendant 5 minutes sont incubées en présence de 3 % d'inuline à des températures variant entre 25°C et 65°C à une densité optique de 5. la figure 2 montre l'influence de la température d'incubation sur le pourcentage d'hydrolyse (en ordonnée) au cours du temps (en abscisse).

La température optimale de réaction est de 55°C. Il est préférable de ne pas dépasser cette température; en effet, l'activité est réduite d'un facteur 5 à 60°C.

D/ Essai de réacteur discontinu d'hydrolyse d'inuline à partir de cellules de Kluyveromyces fragilis, immobilisées sur un support solide.

Après une perméabilisation à 65°C pendant 5 minutes, les cellules de Kluyveromyces fragilis ont été emprisonnées dans des gels d'alginate de calcium de 1 à 2 %.

Les cellules immobilisées sont placées dans une solution d'inuline à 10 % à une température de 55°C (conditions optimales pour l'activité).

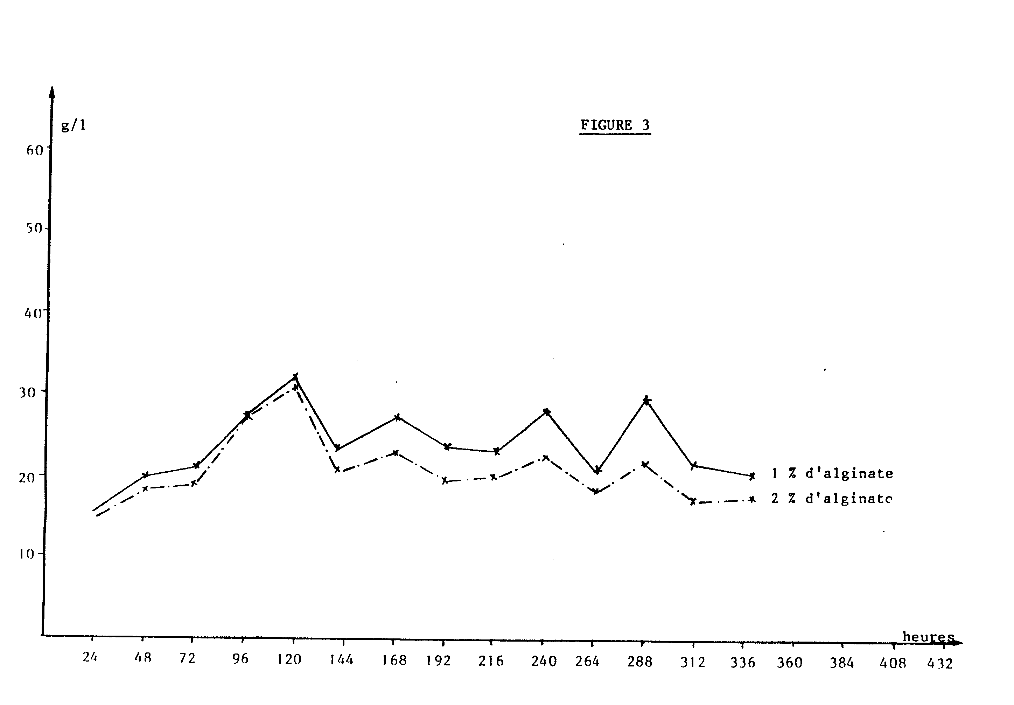

La figure 3 présente l'évolution des réacteurs discontinus à 55°C avec les cellules immobilisées dans des gels d'alginate, exprimée en g/l de solution d'inuline hydrolysée; la solution d'inuline de 10 % est remplacée toutes les 24 heures.

La meilleure efficacité est obtenue avec les billes emprisonnées dans un gel contenant 1 % d'alginate.

Les résultats à 55°C et 10 % d'inuline sont nettement supérieurs à ceux obtenus à 55°C et 3% d'inuline (résultats non représentés sur la figure). Dans ce dernier cas, les billes d'alginate 2 % donnent des résultats semblables à ceux obtenus avec 1 % d'alginate.

Les billes de 2 % présentent cependant, une résistance mécanique plus importante.

Exemple 2

Réacteur discontinu d'hydrolyse d'inuline avec Candida pseudotropicalis IP 513

Les cellules sont produites comme dans l'exemple 1. L'activité inulinase endocellulaire s'élève à 49500 unités/g de cellules. Les cellules sont dénaturées à 60, 65 et 70°C pendant un temps compris entre 5 et 10 minutes pour obtenir une mortalité totale et son incubées à 55°C pendant 24 heures.

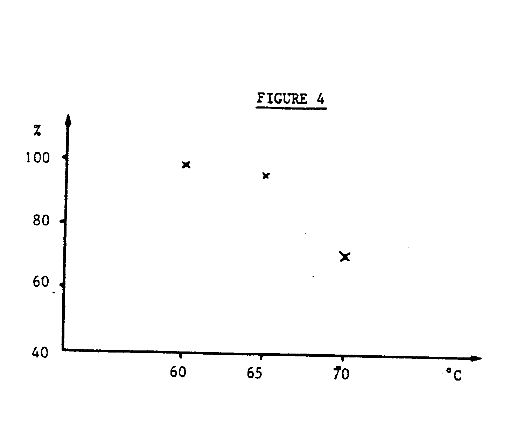

La figure 4 représente le pourcentage d'hydrolyse (en ordonnée) d'une solution à 105 g d'inuline par litre obtenu après 24h à 55°C à pH 4,85 avec les cellules dénaturées à 60, 65 et 70°C (en abscisse).

Les cellules thermodénaturées à 65°C et 60°C présentent une très bonne activité.

Les cellules thermodénaturées à 65°C ont été ensuite immobilisées dans un gel d'alginate 2 % comme décrit précédemment.

20 g de billes contenant 2 % d'alginate de calcium et à une densité cellulaire de 9,25 sont incubées à 55°C, 60°C et 65°C dans un jus d'extraction de carottes de chicon pendant 276 heures.

8

Le tableau 1 reprend les résultats des réacteurs d'hydrolyse d'inuline (concentration 92g/l) à 55, 60 et 65°C.
Ces résultats sont exprimés en % d'hydrolyse (colonne A) et en g/l de réacteur/24 h (colonne B).
Les réacteurs donnent de bons résultats à des températures inférieures à 65°C et ces résultats démontrent la faisabilité du procédé avec d'autres souches que Kluyveromyces.

TABLEAU I

| Période cumulée (h) | Durée du réacteur discontinu (h) | Température d'incubation | | | | | |
|---|---|---|---|---|---|---|---|
| | | 55°C | | 60°C | | 65°C | |
| | | A | B | A | B | A | B |
| 24 | 24 | 51 | 56 | 50 | 55 | 25 | 32 |
| 96 | 72 | 88 | 29 | 79 | 26 | 13 | 13 |
| 120 | 24 | 72 | 74 | 58 | 62 | 9 | 19 |
| 140 | 20 | 67 | 84 | 52 | 68 | 4 | 18 |
| 164 | 24 | 67 | 70 | 51 | 58 | 2 | 13 |
| 184 | 20 | 69 | 86 | 50 | 67 | 2 | 16 |
| | | 66 | 23 | 48 | 18 | 2 | 4 |
| 256 | 72 | 47 | 63 | 18 | 33 | 1 | 15 |
| 276 | 20 | 50 | 47 | 14 | 13 | 0,1 | 0,1 |

Exemple 3
Réacteur continu d'hydrolyse d'inuline avec Kluyveromyces fragilis immobilisée dans un gel d'alginate.
Un réacteur continu d'hydrolyse d'inuline a été réalisé afin de tester sa viabilité et de mesurer l'efficacité du procédé en ce qui concerne la longévité.
Les caractéristiques de la colonne sont les suivantes :
- 200 ml de billes d'alginate, 2% d'alginate, 30 de densité optique cellulaire, t° : 55°C et la concentration en inuline dans la solution d'alimentation est de 10 %.

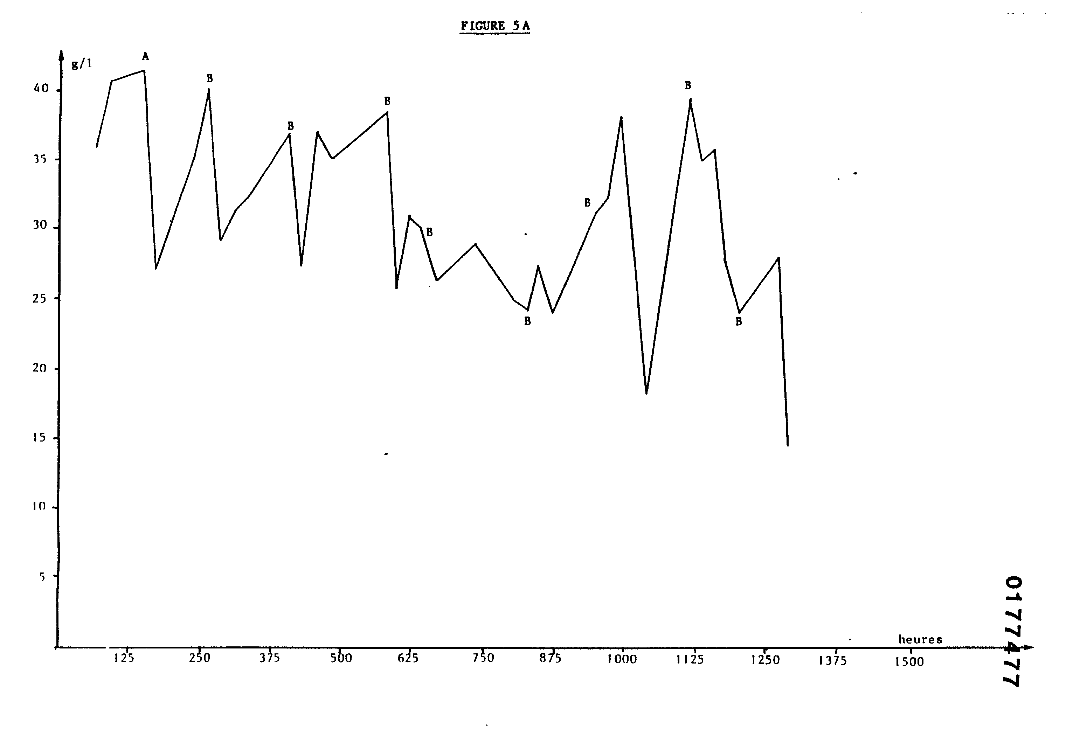

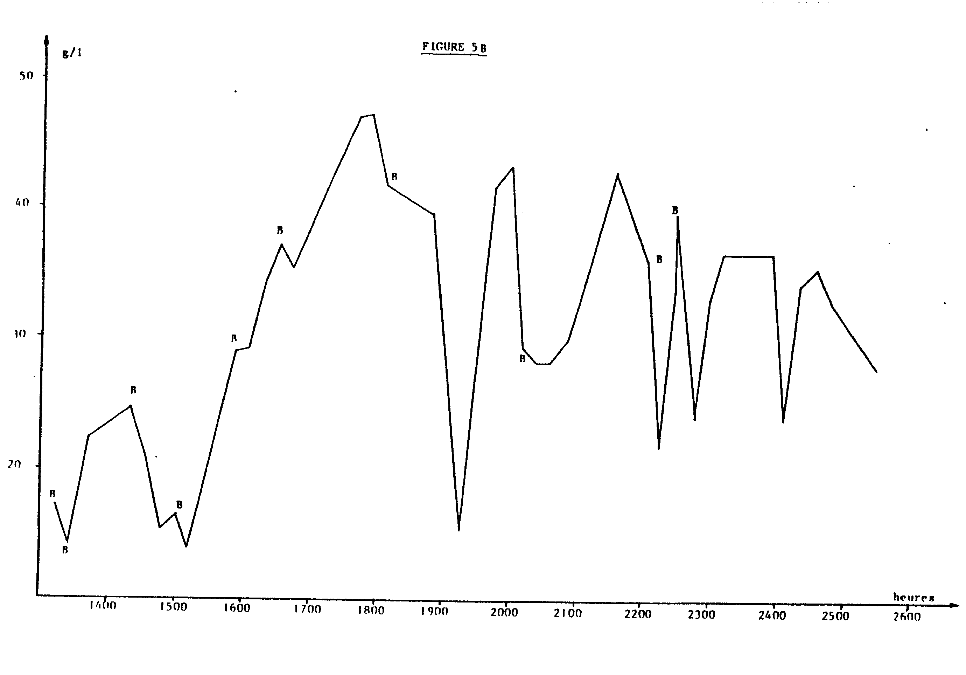

Les figures 5A et 5B reprennent l'évolution du réacteur d'hydrolyse d'inuline exprimée en g/l de solution respectivement de 0 à 1.300 heures et de 1.300 à 2.600 heures de fonctionnement.

Au début du fonctionnement du réacteur, celui-ci a été lavé avec une solution de $CaCl_2$ (A);on note une diminution importante de l'activité mais ensuite une reprise de cette activité. Ce fait est probablement dû à la perturbation des phénomènes de transferts de l'inuline par ce lavage. Les lavages suivants (B) ont été effectués avec de l'eau distillée et des solutions d'inuline.

Exemple 4

Réacteur d'hydrolyse d'inuline par des cellules de Kluyveromyces fragilis immobilisées par adsorption sur sciure de peuplier.

Une colonne de 6 grammes de sciure de peuplier (12-18 mesh) est alimentée par une suspension de cellules de Kluyveromyces fragilis à une densité optique de 10,4 (soit 3,9 mg de cellules par ml dans le tampon acétate 0,05M,pH 4,85). Les cellules de Kluyveromyces ont été cultivées comme décrit précédemment et thermodénaturées durant 5 minutes à 65°C. Dans ces conditions,toutes les cellules sont tuées (test au bleu de méthylène).

A la suspension cellulaire, 300 ppm d'amidon cationique sont ajoutés afin d'obtenir un potentiel zéta des cellules de + 3,5. La quantité de cellules fixées est de 204mg/g de support.

La colonne est ensuite alimentée à 55°C par une solution de 10 % d'inuline durant 500 heures; le réacteur hydrolyse ± 90 g d'inuline par litre de réacteur et par 24 h pendant au moins 500 heures.

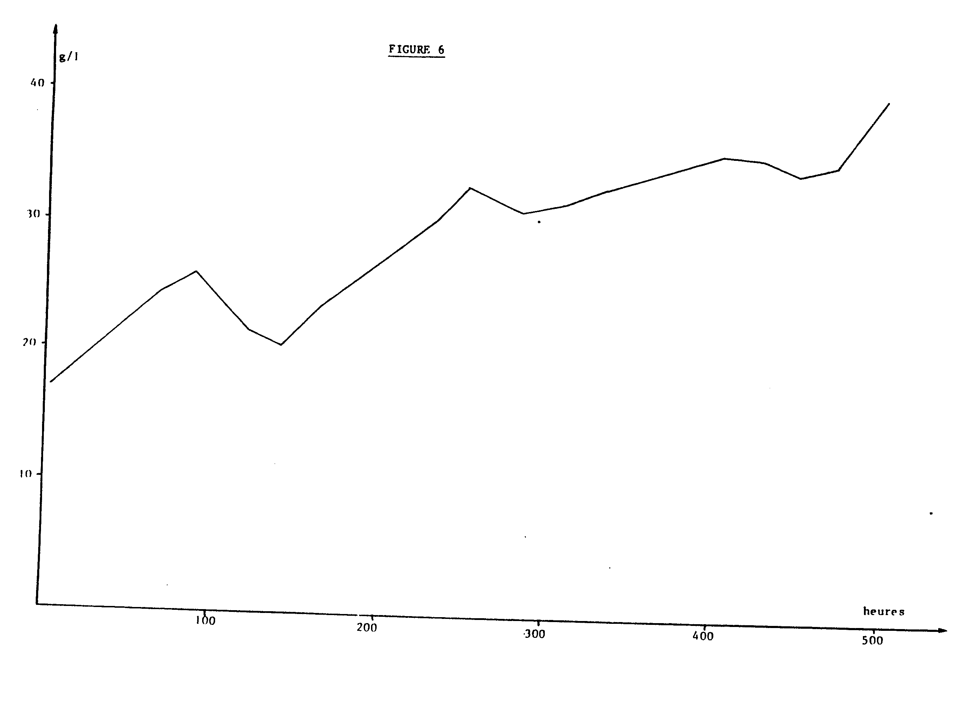

La figure 6 donne l'évolution de l'hydrolyse continue d'une solution d'inuline à 55°C par des cellules adsorbées sur la sciure de peuplier (exprimée en g/l de solution).

Cette expérience montre que le procédé d'immobilisation influence le rendement du réacteur. L'immobilisation dans un réacteur de billes d'alginate est 2 fois moins performant (g/l/24 heures) mais est plus facile à laver lors d'infection.

0177477

REVENDICATIONS

1. Procédé d'hydrolyse enzymatique de solutions aqueuses d'inuline par des cellules microbiennes possédant une activité inulinase, caractérisé en ce que les cellules microbiennes sont soumises à un traitement thermique de perméabilisation des parois cellulaires à une température et pendant une durée telles que toutes les cellules sont tuées.

2. Procédé d'hydrolyse enzymatique de solutions aqueuses d'inuline par des cellules microbiennes possédant une activité inulinase, suivant la revendication 1, caractérisé en ce que le traitement thermique de perméabilisation des parois cellulaires des cellules microbiennes s'effectue à une température de 55°C à 75°C pendant une durée pouvant atteindre 15 minutes.

3. Procédé d'hydrolyse enzymatique de solutions aqueuses d'inuline par des cellules microbiennes possédant une activité inulinase, suivant les revendications 1 et 2, caractérisé en ce que le traitement thermique de perméabilisation des parois cellulaires des cellules microbiennes s'effectue à une température de 55°C à 75°C pendant une durée comprise entre 5 et 10 minutes.

4. Procédé d'hydrolyse enzymatique de solutions aqueuses d'inuline par des cellules microbiennes possédant une activité inulinase, suivant les revendications 1 à 3, caractérisé en ce que les cellules microbiennes proviennent de levures, de moisissures ou de bactéries.

5. Procédé d'hydrolyse enzymatique de solutions aqueuses d'inuline par des cellules microbiennes possédant une activité inulinase, suivant les revendications 1 à 4, caractérisé en ce que les cellules microbiennes provenant de levures sont choisies parmi les souches de Kluyveromyces, de Saccharomyces, de Candida et de Pichia.

6. Procédé d'hydrolyse enzymatique de solutions aqueuse d'inuline par des cellules microbiennes possédant une activité inulinase suivant les revendications 1 à 4, caractérisé en ce que les cellules microbiennes provenant de moisissures et de bactéries sont choisies parmi les souches d'Aspergillus, de Penicillium, de Fusarium, d'Achromobacter et de Acetobacter.

7. Procédé d'hydrolyse enzymatique de solutions aqueuses d'inuline par des cellules microbiennes possédant une activité inulinase, suivant les revendications 1 à 6, caractérisé en ce que les cellules

microbiennes sont immobilisées sur un support solide et sont soumies avant ou après immobilisation à un traitement thermique de perméabilisation à une température et pendant une durée telles que toutes les cellules sont tuées.

FIGURE 1

0177477

FIGURE 2

FIGURE 3

1 % d'alginate

2 % d'alginate

g/1

heures

0177477

FIGURE 4

FIGURE 5 A

FIGURE 5 B

FIGURE 6

0177477